# EUROPEAN PATENT SPECIFICATION

(11) **EP 1 620 549 B1**
(45) Date of publication and mention of the grant of the patent: **22.08.2007**
(21) Application number: 04730939.8
(22) Date of filing: 03.05.2004
(51) Int. Cl.: C12N 1/02, C12P 1/00, C12M 3/00, C12N 5/00

(54) **PROCESS FOR THE PRODUCTION OF BIOLOGICAL SUBSTANCES BY PERFUSION CULTURING OF SUSPENDED ANIMAL CELLS**
VERFAHREN ZUR HERSTELLUNG VON BIOLOGISCHEN SUBSTANZEN DURCH PERFUSIONSKULTIVIERUNG VON SUSPENDIERTEN TIERISCHEN ZELLEN
PROCEDE POUR LA PRODUCTION DES SUBSTANCES BIOLOGIQUES PAR CULTURE A PERFUSION DE CELLULES ANIMALES SUSPENDUES

(30) Priority: 01.05.2003 EP 03101205
(43) Date of publication of application: 01.02.2006
(73) Proprietor: DSM IP Assets B.V., 6411 TE Heerlen (NL)
(72) Inventor: POL, VAN DER, Leonardus, Adolfus, NL-9717 AS Groningen (NL)
(74) Representative: Breepoel, Peter M.
(86) International application number: PCT/NL2004/000300
(87) International publication number: WO 2004/097006

(56) References cited:
- US-A- 5 372 943
- US-A- 5 378 612
- SAKAI KENTARO ET AL: "Use of nonionic surfactants for effective supply of phosphatidic acid in serum-free culture of Chinese hamster ovary cells." JOURNAL OF BIOSCIENCE AND BIOENGINEERING, vol. 92, no. 3, 2001, pages 256-261, XP002255475 ISSN: 1389-1723
- MICHIYUKI TOKASHIKI ET AL: "HIGH DENSITY CULTURE OF HYBRIDOMA CELLS USING A PERFUSION CULTURE VESSEL WITH AN EXTERNAL CENTRIFUGE" CYTOTECHNOLOGY, KLUWER ACADEMIC PUBLISHERS, DORDRECHT, NL, vol. 3, no. 3, 1 May 1990 (1990-05-01), pages 239-244, XP000117122 ISSN: 0920-9069

## Description

The invention relates to a process for the production of biological substances by perfusion culturing of suspended animal cells in a serum free cell culture medium, wherein the biological substance is separated from the cells by filtration.

Such processes are known in the art, for instance a review on perfusion culturing was written by Tokashiki et al. 1993. Cytotechnology, vol. 13: 149-159. In perfusion culturing, wherein the biological substance is separated from the cells by filtration, it is preferred that the filtration is such that 1) the cells are retained by the filter, while 2) liquid comprising the biological substance passes through the filter. A measure for how well a filter performs these two tasks is the product of the mean total cell concentration in the cell culture and the total volume that passes the filter (filter performance). The filter performance is proportional to the yield of the biological substance.

A disadvantage of perfusion culturing, wherein the biological substance is separated from the cells by filtration, however, is that filter clogging occurs during the process. Filter clogging limits the total amount of volume that can pass the filter, thereby limiting filter performance and thereby also limiting the yield of the biological substance.

It is the object of the invention to provide a process for the production of biological substances by perfusion culturing of suspended animal cells in a serum free cell culture medium, wherein the biological substance is separated from the cells by filtration, wherein filter clogging is diminished (i.e. the filter performance is increased).

This object is achieved by the invention by the presence of an amount of non-ionic surfactant of at least 0.001 w/w% in the cell culture medium.

The use of non-ionic surfactants in a serum-free cell culture medium for mammalian cells is known from US 5,372,943, however, in US 5,372,943, the use is directed to the provision of essential lipids in a bioavailable form in the cell culture medium. The applicant has surprisingly found that the use of non-ionic surfactants in a serum-free culture medium for mammalian cells also diminishes filter clogging.

In principle, the upper limit of the amount of non-ionic surfactant to be used in the serum-free culture medium is not very critical. It may, however, for example be limited by the toxicity level and/or the solubility of the non-ionic surfactant. The upper limit further may depend on the process conditions and can easily be determined by the person skilled in the art. Usually, the upper limit of the amount of non-ionic surfactant present in the cell culture medium is 1 w/w %.

In the process of the invention, the non-ionic surfactant is preferably a fatty acid ester, for instance a fatty acid ester of glycerol or of diglycerol or a polyoxyalkylene sorbitan fatty acid ester represented by formula 1, 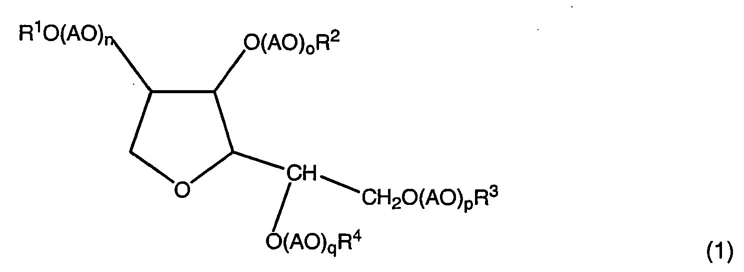 wherein R¹, R², R³ and R⁴ each independently represent H or a fatty acid restgroup; i.e. the remains of a condensation of a fatty acid and an alcohol, provided that at least one of R¹ through R⁴ is a fatty acid restgroup, wherein A represents an ethylene or propylene group and n, o, p and q each independently represent values from 0 to 100, wherein preferably the sum of n, o, p, and q is from 50 to 300.

Preferably the fatty acid restgroup contains 10-20 C-atoms. Examples of fatty acids include decanoic acid, lauric acid, palmitic acid, stearic acid, arachidic acid, tall oil acids etc.

Examples of commercially available compounds of formula 1 include Tween^{™}-compounds, e.g. Tween^{™}-80, Tween^{™}-21, Tween^{™}-40, Tween^{™}-60, Tween^{™}-20, Tween^{™}-61, Tween^{™}-65, Tween^{™}-81, Tween^{™}-85. Of course, it is also possible to use a combination of different non-ionic surfactants in the process of the invention.

The fatty acid esters are especially advantageous in the process of the invention, since the presence of small amounts of these esters does not present an obstacle for use of the biological substance in a medical application.

Preferably, the amount of the noniononic surfactant in the cell culture medium is at least 0.005, more preferably at least 0.01, most preferably at least 0.02 w/w%.

The animal cells that can be used in the process of the present invention are for example mammalian cells, for example CHO (Chinese Hamster Ovary) cells, hybridomas, BHK (Baby Hamster Kidney) cells, myeloma cells and human cells, for example HEK-293 cells and human lymphoblastoid cells.

The filtration can in principle be performed with all filters suitable for the retention of cells. Examples of such filters are static filters, for example hollow fiber filter or tangential flow filters or rotating filters, for example spinfilters. Preferably, if the biological substance is a biopharmaceutical product, an internal filter is used as the use of an external filter requires additional validation for the approval of the production process of the biopharmaceutical product according to GMP (good manufacturing practice) standards. An example of a suitable internal filter is an internal spinfilter.

The most suitable mesh size of a filter depends on the cell diameter of the cells to be retained and can easily be determined by a person skilled in the art. The mesh size is preferably chosen such that the size of the pores in the mesh is close to the diameter of the suspended cells, ensuring a high retention of cells while cell debris can pass the filter. Preferably, the meshsize is between 500x3000-50x300.

The invention can in principle be used in any type of cell culture medium suitable for the culturing of animal cells. Guidelines for choosing a cell culture medium and cell culture conditions are well known and are for instance provided in Chapter 8 and 9 of Freshney, R. I. Culture of animal cells (a manual of basic techniques), 4th edition 2000, Wiley-Liss and in Doyle, A., Griffiths, J. B., Newell, D. G. Cell &Tissue culture: Laboratory Procedures 1993, John Wiley & Sons.

Serum free media are preferred to media containing a serum source in the production of biopharmaceutical products as serum source media are frequently contaminated with viruses, present the risk of prionic infections, and can create a major obstacle in the downstream processing of the biopharmaceutical product (i.e. the further purification of the biopharmaceutical product from the cell culture medium). Since compounds from a mammalian source also present an infection risk, preferably, the cell culture medium is not only serum-free, but also mammalian source free. More preferably the cell culture medium is not only mammalian source-free, but also animal source free.

The pH, temperature, dissolved oxygen concentration and osmolarity of the cell culture medium are in principle not critical and depend on the type of cell chosen. Preferably, the pH, temperature, dissolved oxygen concentration and osmolarity are chosen such that it is optimal for the growth and productivity of the cells. The person skilled in the art knows how to find the optimal pH, temperature, dissolved oxygen concentration and osmolarity for the perfusion culturing. Usually, the optimal pH is between 6.6 and 7.6, the optimal temperature between 30 and 39°C, the optimal osmolarity between 260 and 400mOsm and the dissolved oxygen concentration in the cell culture medium between 5 and 95% of air saturation.

The biological substances that can suitably be produced in the perfusion culturing by the animal cell are in principle all biological substances that can be produced by an animal cell, for example therapeutic and diagnostic proteins, for example monoclonal antibodies, growth factors and enzymes, DNAs, which for example might be used in gene therapy, vaccines, hormones etc. Preferably, the process according to the invention is used for the production of a biopharmaceutical product, which is a biological substance with a medical application. Examples of biopharmaceutical products are as follows (with examples of brand names of the corresponding biopharmaceutical product between brackets): Tenecteplase (TN Kase^{™}), (recombinant) antihemophilic factor (ReFacto^{™}), lymphoblastoid Interferon α-n1 (Wellferon^{™}), (recombinant) Coagulation factor (NovoSeven^{™}), Etanercept, (Enbrel^{™}), Trastuzumab (Herceptin^{™}), Infliximab (Remicade^{™}), Basiliximab (Simulect^{™}), Daclizumab (Zenapaz^{™}), (recombinant) Coagulation factor IX (Benefix^{™}), erythropoietin alpha (Epogen®), G-CSF (Neupogen®Filgrastim), Interferon alpha-2b (Infergen®), recombinant insulin (Humulin®), Interferon beta 1a (Avonex®), Factor VIII (KoGENate®), Glucocerebrosidase (Cerezyme^{™}), Interferon beta 1b (Betaseron®), TNF alpha receptor (Enbrel®), Follicle stimulating hormone (Gonal-F®), Mab abcixmab (Synagis®, ReoPro®), Mab ritiximab (Rituxan®), tissue plasminogen activator (Activase®, Actilyase®), human growth hormone (Protropin®, Norditropin®, GenoTropin^{™}). Examples of DNAs with a possible medical application are gene therapeutic plasmid DNAs. Some gene therapeutic DNAs are presently tested in clinical trials for their medical application. Examples of vaccines are live, oral, tetravalent Rotavirus vaccine (RotaShield^{™}), rabies vaccine (RanAvert^{™}), Hepatitis B vaccin (RECOMBIVAX HB®, Engerix®) and inactivated hepatitis A vaccine (VAQTA^{™}).

The biological substance, which was passed through the filter can be further purified in so-called downstream processing. Downstream processing usually comprises several purification steps in varying combinations and order. Examples of purification steps in the downstream processing are separation steps (e.g. by affinity chromatography and/or ion exchange chromatography), steps for the concentration of the biological substance (e.g. by ultrafiltration or diafiltration), steps to exchange buffers and/or steps to remove or inactivate viruses (e.g. by virusfiltration, pH shift or solvent detergent treatment).

The invention will be elucidated by way of the following example, without, however, being limited thereto.

### Example I

PFU-83 hybridoma cells are rat/mouse hetero hybridoma cells producing a rat IgG directed against corticotropin releasing factor (J. W. A. M. van Oers et al. 1989, Endocrinology, 124, pp1239-1246). PFU-83 hybridoma cells were grown on minimal mammalian source free medium at 37°C. The minimal mammalian source free medium used was 3:1 DMEM/Ham's F12 medium, containing ascorbic acid (5 mg/l), recombinant human insulin (5 mg/l), glutathion (1 mg/l), Pluronic^{™} F68 (500 mg/l), selenium in the form of Na₂SeO₃ (15µg/l), sodium bicarbonate (3 g/l), ethanolamine (7µl/l), supplemented with glutamin (final concentration 584.5 mg/l), Na-pyruvate (final concentration 110 mg/l) and transferrin (5 mg/l) with an osmolality of 320 mOsm. The pH was set at 7.0 and the dissolved oxygen concentration was set at 30% air saturation. The culture was performed in a 7 I Applikon fermentor running at 4 I working volume using a spin filter with a mesh size of 325 x 2300 in the presence of different concentrations of Tween®-80 (0, 15 mg/l and 50 mg/l ; corresponding to approximately 0; 0.015; and 0.050 w/w%). A viable cell concentration of approximately 5*10⁶ was reached after 4 to 5 days.

All different runs had a cell retention of 100%. The time until complete clogging of the filter (the point when the spinfilter flooded) was observed and the spin filter performance was calculated. Spin filter performance is defined as the product of the total volume that passed the spinfilter and the mean total cell concentration in the fermentor. Cell concentrations in the fermentor were determined by counting the total amount of viable and dead cells in cell culture samples taken during the run of the fermentor under a light microscope using a Fuchs-Rosenthal haemacytometer and trypan-blue exclusion for staining of dead cells. The results of this experiment are shown in Table 1 below. From Table 1 it can be seen that the time until clogging, as well as the spin filter performance, is increased with the addition of Tween®-80.

**Table 1**

| [Tween®-80] (mg/l) | Time until complete clogging (days) | Total volume that passed the spinfilter (l) | Mean total cell concentration (x 10⁹/l) | Spin filter performance (total cell load) |
|---|---|---|---|---|
| 0 | 7 | 14 | 3.77 | 53 |
| 15 | 9 | 25 | 4.97 | 127 |
| 50 | 29 | 99 | 4.68 | 463 |

## Claims

1. Process for the production of a biological substance by perfusion culturing of suspended animal cells in a serum free cell culture medium, wherein the biological substance is separated from the cells by filtration, **characterized in that** at least 0.001 w/w% of polyoxyalkylene sorbitan fatty acid ester represented by formula 1, 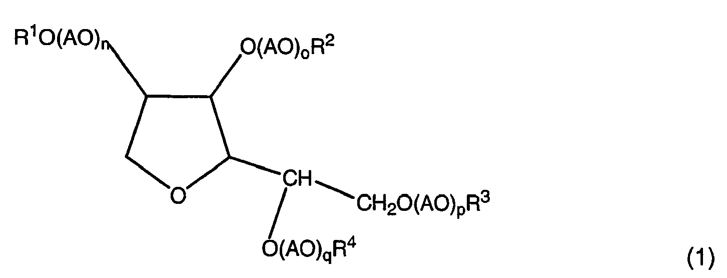 wherein R¹, R², R³ and R⁴ each independently represent H or a fatty acid restgroup, i.e. the remains of a condensation of a fatty acid and an alcohol, provided that at least one of R¹ through R⁴ is a fatty acid restgroup, wherein A represents an ethylene or propylene group and n, o, p and q each independently represent values from 0 to 100, is present in the cell culture medium.

2. Process according to claim 1, **characterized in that** in formula 1, the sum of n, o, p, and q is from 50 to 300.

3. Process according to claim 1 or claim 2, **characterized in that** at least 0.01 w/w% of the compound of formula 1 is present in the cell culture medium.

4. Process according to any of claims 1-3, **characterized in that** the animal cells are mammalian cells.

5. Process according to any of claims 1-4, **characterized in that** the compound of formula 1 is a Tween^{™} compound.

6. Process according to any of claims 1-5, **characterized in that** the filtration is performed with an internal filter.

7. Process according to claim 6, **characterized in that** the internal filter is a spinfilter.

8. Process according to any of claims 1-7, **characterized in that** the biological substance is a biopharmaceutical product.

9. Process according to claim 8, **characterized in that** the serum free cell culture medium is also a mammalian source free medium.

10. Process according to any of claims 1-9, **characterized in that** the biological substance is further purified by downstream processing.

## Patentansprüche

1. Verfahren zur Produktion einer biologischen Substanz durch Perfusionskultivierung suspendierter Tierzellen in einem serumfreien Zellkulturmedium, wobei die biologische Substanz von den Zellen mittels Filtration abgetrennt wird, **dadurch gekennzeichnet, dass** mindestens 0,001 Gew.-% Polyoxyalkylensorbitanfettsäureester der Formel 1 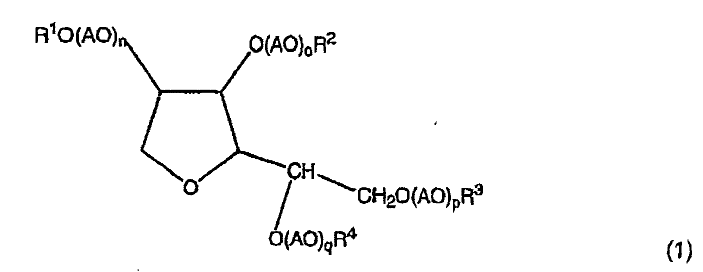 wobei R¹, R², R³ und R⁴ jeweils unabhängig für H oder eine Fettsäurerestgruppe, d.h. den Überrest aus einer Kondensation einer Fettsäure und eines Alkohols, stehen, mit der Maßgabe, dass mindestens einer der Reste R¹ bis R⁴ eine Fettsäurerestgruppe ist, wobei A für eine Ethylen- oder Propylengruppe steht und n, o, p und q jeweils unabhängig für Werte von 0 bis 100 stehen, im Zellkulturmedium vorhanden ist.

2. Verfahren nach Anspruch 1, **dadurch gekennzeichnet, dass** in Formel 1 die Summe von n, o, p und q von 50 bis 300 reicht.

3. Verfahren nach Anspruch 1 oder Anspruch 2, **dadurch gekennzeichnet, dass** mindestens 0,01 Gew.-% der Verbindung der Formel 1 im Zellkulturmedium vorhanden ist.

4. Verfahren nach einem der Ansprüche 1 bis 3, **dadurch gekennzeichnet, dass** die Tierzellen Säugerzellen sind.

5. Verfahren nach einem der Ansprüche 1 bis 4, **dadurch gekennzeichnet, dass** die Verbindung der Formel 1 eine Tween^{™}-Verbindung ist.

6. Verfahren nach einem der Ansprüche 1 bis 5, **dadurch gekennzeichnet, dass** die Filtration mit einem Innenfilter durchgeführt wird.

7. Verfahren nach Anspruch 6, **dadurch gekennzeichnet, dass** der Innenfilter ein Spinfilter ist.

8. Verfahren nach einem der Ansprüche 1 bis 7, **dadurch gekennzeichnet, dass** die biologische Substanz ein biopharmazeutisches Produkt ist.

9. Verfahren nach Anspruch 8, **dadurch gekennzeichnet, dass** das serumfreie Zellkulturmedium auch ein von einer Säugerquelle freies Medium ist.

10. Verfahren nach einem der Ansprüche 1 bis 9, **dadurch gekennzeichnet, dass** die biologische Substanz mittels Downstream-Processing weiter gereinigt wird.

## Revendications

1. Procédé de production d'une substance biologique par culture sous perfusion de cellules animales en suspension dans un milieu de culture cellulaire exempt de sérum, dans lequel la substance biologique est séparée des cellules par filtration, **caractérisé en qu'**au moins 0,001 % p/p d'ester d'acide gras de polyoxyalkylène sorbitane représenté par la formule 1, 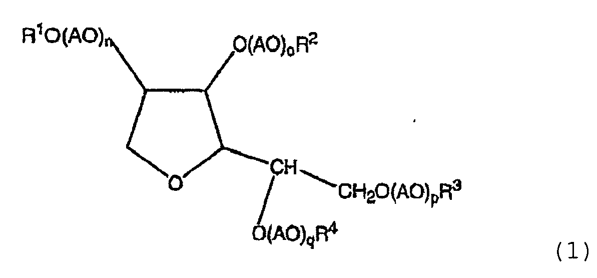 dans laquelle R¹, R², R³ et R⁴ représentent chacun indépendamment un atome H ou un groupe restant d'acide gras, c'est-à-dire les restes d'une condensation d'un acide gras et d'un alcool, à condition qu'au moins un de R¹ à R⁴ soit un groupe restant d'acide gras, dans laquelle A représente un groupe éthylène ou propylène et n, o, p et q représentent chacun indépendamment des valeurs de 0 à 100, est présent dans le milieu de culture cellulaire.

2. Procédé selon la revendication 1, **caractérisé en ce que** la somme de n, o, p et q dans la formule 1 varie de 50 à 300.

3. Procédé selon la revendication 1 ou 1a revendication 2, **caractérisé en ce qu'**au moins 0,01 % p/p du composé de formule 1 est présent dans le milieu de culture cellulaire.

4. Procédé selon l'une quelconque des revendications 1 à 3, **caractérisé en ce que** les cellules animales sont des cellules de mammifère.

5. Procédé selon l'une quelconque des revendications 1 à 4, **caractérisé en ce que** le composé de formule 1 est un composé Tween^{™}.

6. Procédé selon l'une quelconque des revendications 1 à 5, **caractérisé en ce que** la filtration est effectuée à l'aide d'un filtre interne.

7. Procédé selon la revendication 6, **caractérisé en ce que** le filtre interne est un filtre rotatif.

8. Procédé selon l'une quelconque des revendications 1 à 7, **caractérisé en ce que** la substance biologique est un produit biopharmaceutique.

9. Procédé selon la revendication 8, **caractérisé en ce que** le milieu de culture cellulaire exempt de sérum est également un milieu exempt d'une source mammifère.

10. Procédé selon l'une quelconque des revendications 1 à 9, **caractérisé en ce que** la substance biologique est en outre purifiée par un procédé en aval.
